# EUROPEAN PATENT SPECIFICATION

(11) **EP 3 137 084 B1**
(45) Date of publication and mention of the grant of the patent: **25.12.2019**
(21) Application number: 15786354.9
(22) Date of filing: 01.05.2015
(51) Int. Cl.: A61K 31/4965, A61K 45/06, A61P 17/06

(54) **BENZYL AMILORIDE FOR THE TREATMENT OF PSORIASIS**
BENZYL AMILORID ZUR BEHANDLUNG VON PSORIASIS
AMILORIDE DE BENZYLE POUR LE TRAITEMENT DU PSORIASIS

(30) Priority: 02.05.2014 US 201461987691 P
(43) Date of publication of application: 08.03.2017
(73) Proprietor: The Board of Trustees of the Leland Stanford Junior University, Stanford, CA 94305-2038 (US); U.S. Government Represented by the Department of Veteran Affairs, Washington DC 20420 (US)
(72) Inventor: MARINKOVICH, Peter M., Redwood City, California 94061 (US); BUTTE, Atul J., Menlo Park, California 94025 (US); NASRALLAH, Mazen, Cambridge, MA 02141 (US); WINGE, Carl Gustaf Maarten, Mountain View, California 94040 (US)
(74) Representative: Mewburn Ellis LLP
(86) International application number: PCT/US2015/028820
(87) International publication number: WO 2015/168574

(56) References cited:
- WO-A1-02/085367
- JP-A- 2006 232 684
- US-A1- 2004 087 480
- US-A1- 2006 127 318
- US-A1- 2007 161 543
- US-A1- 2011 280 845
- US-A1- 2014 011 817
- US-B1- 6 264 975
- US-B2- 6 903 105
- RAHMAN M ET AL: "Classical to current approach for treatment of psoriasis: a review", ENDOCRINE, METABOLIC & IMMUNE DISORDERS - DRUG TARGETS, BENTHAM SCIENCE PUBLISHERS LTD, BUSSUM, NL, vol. 12, no. 3, 1 September 2012 (2012-09-01), pages 287-302, XP002742331, ISSN: 1871-5303, DOI: 10.2174/187153012802002901
- SIMONA FRATESCHI ET AL: "The Epithelial Sodium Channel ENaC and its Regulators in the Epidermal Permeability Barrier Function", THE OPEN DERMATOLOGY JOURNAL, vol. 4, no. 1, 1 January 2010 (2010-01-01) , pages 27-35, XP055422684, NL ISSN: 1874-3722, DOI: 10.2174/1874372201004010027

## Description

### BACKGROUND OF THE INVENTION

Psoriasis is an immune-mediated skin disease appearing in a chronic recurring manner. Prevalence estimates show that it affects 1-2% of the worldwide population with equal gender distribution. Psoriasis can emerge at any time of life and it usually peaks between the ages of 30-39 and 60-69. Sufferers may experience itch, pain, and/or psoriasis-related nail disease and arthritis. Significant morbidity extends to the psychosocial impact on the individual. Psoriatic patients are often stigmatized by people staring at their disfigured skin; they may have low self-esteem and would face difficulties in relationships and employment. Psoriasis has also been associated with an increased risk of cardiovascular diseases, stroke and cancer.

Histological assessment of psoriatic plaques demonstrates keratinocyte hyperproliferation with parakeratosis, epidermal elongation or rete ridges, increased angiogenesis, and dermal infiltration of inflammatory cells, including T cells, neutrophils, macrophages, and dendritic cells (DCs). Other histological features often observed in psoriatic skin include micropustules of Kogoj, microabscesses of Munro, thinned or absent granular layer, thinned suprapapillary plates, and the papillary dermis containing dilated superficial vessels.

The etiology of psoriasis is multifactorial. Environmental triggers, such as trauma, stress, infections, and drugs, activate in predisposed individuals an exaggerated inflammatory response in the skin. Although psoriasis is a disease of dysfunctional proliferation and differentiation of the keratinocytes, there is significant T cell involvement through the release of inflammatory cytokines that promote further recruitment of immune cells, keratinocyte proliferation, and sustained chronic inflammation. These T-cells proliferate in the epidermis of psoriatic plaques.

The presence of innate immune cells and their products in psoriatic skin plaques indicates a role for innate immunity. Cells of the innate immune system include macrophages, NK and NKT cells, and DCs. There is an increased number of plasmacytoid and myeloid DCs in psoriatic skin compared with non-lesional skin. Other cellular elements of innate immunity are also involved in the development of psoriasis, including high numbers of macrophages which can secrete IL-6, IL-12, IL-23, and TNF-a. Keratinocytes are also capable resident antigen-presenting cells (APCs) in the skin. When stimulated they produce large amounts of cytokines (e.g., TNF-a, IL-6, and IL-18), chemotactic chemokines (e.g., IL-8 and CCL20), and antimicrobial peptides (e.g., β-defensin and LL37).

Genome wide scans have reported at least nine chromosomal loci linked to psoriasis. PSORS1 accounts for 35-50% of the heritability of the disease. PSORS1 is located on the major histological complex (MHC) region of chromosome 6 (6p21). Three genes contained within this region are associated with psoriasis, namely, HLA-Cw6, CCHCR1 (coiled-coil α-helical rod protein), and CDSN (corneodesmosin). Other susceptibility loci have been identified which include genes expressed in keratinocytes (LCE3B (late cornified envelope 3B) and LCE3C1 (late cornified envelope 3C1)) and immune cells (IL-12B, IL23R, and IL23A), and they are involved in maintaining epidermal skin barrier and immune responses against pathogens.

Currently the first line of treatment for psoriasis is the use of topical agents. When topical therapy fails, escalated treatment often includes phototherapy, oral systemic agents, and/or injectable biological therapies. Corticosteroids, vitamin D analogues, and tazarotene all are used in the treatment of chronic plaque psoriasis. However, prolonged exposure to topical corticosteroids may lead to atrophy of the skin, permanent striae, and telangiectasia. Vitamin D analogues (e.g., calcitriol, calcipotriol, and tacalcitol) are effective antipsoriatic agents, but excessive use can lead to hypercalcaemia. The probability of treatment success doubles when combining vitamin D analogues with topical corticosteroids as compared with the vitamin D analogue monotherapy. As a result, the currently recommended first-line induction treatment of plaque psoriasis is a combination of a vitamin D analogue and a topical steroid.

Other topical agents are commonly combined with topical corticosteroids and vitamin D analogues when treating psoriatic plaques. Salicylic acid is a topical keratolytic agent used adjunctly for removing scales, and it acts by reducing coherence between keratinocytes, increasing hydration, and softening of the stratum corneum by decreasing the skin pH, however, systemic salicylic acid toxicity can occur after long-term use over large skin areas. Retinoids, another popular treatment agent for psoriasis, act on skin by mediating cell differentiation and proliferation. Systemic retinoids, e.g. Tazarotene, are associated with several adverse effects including teratogenicity, serum lipid elevations, mucocutaneous toxicity, skeletal changes, and hair loss.

Ultraviolet (UV) light therapy induces T-lymphocyte apoptosis in psoriatic lesions of the dermis and epidermis. Oral 8-methoxypsoralen-UV-A (PUVA) and narrowband UVB (NB-UVB) are well-established and effective treatments for chronic plaque psoriasis. PUVA has a response rate of approximately 80% compared with 70% for NB-UVB, however, NB-UVB is preferred because of higher convenience, except in case of very thick plaques.

Systemic treatments are often used in combination with topical therapy and phototherapy for patients with severe psoriasis. Oral systemic agents for the treatment of psoriasis include methotrexate, cyclosporine, and acitretin. Injectable biological therapies are emerging approaches for the treatment of psoriasis by targeting molecules in the inflammatory pathways. They are considered for patients with severe psoriasis that are resistant to oral immunosuppressants and phototherapy. The two major therapeutic classes of injectable biological therapies include anti-cytokine therapies and T-cell-targeted therapies. The first class consists of injectable immunoglobulins (Ig), infliximab, and adalimumab, target soluble and membrane-bound TNF-a. Other anti-cytokine therapies include Etanercept and Ustekinumab. A second therapeutic class of injectable therapies include agents that bind to T cells and prevent T-cells activation, including alefacept and efalizumab.

Dermatologists and patients would benefit from new therapies for psoriasis, particularly those that can be delivered topically.

### SUMMARY OF THE INVENTION

Provided herein are compositions for use in methods of treating psoriasis according to the claims. In the methods disclosed herein, an effective dose of an epithelial ion channel (ENAC) blocker is administered to an individual suffering from psoriasis, where the dose is effective for reducing the Psoriasis Area and Severity Index of the individual. Applicants demonstrate that ENAC blockers, including without limitation Benzamil, can reduce thickened cutaneous psoriatic plaques.

ENAC blockers of interest include, without limitation, triamterene, amiloride and derivatives, and the like. In the present invention the ENAC blocker used is benzyl amiloride (Benzamil).

In some disclosures the ENAC blocker is systemically administered, e.g. by i.p., i.m., i.v. injection, *etc.* In other disclosures the ENAC blocker is topically administered, e.g. formulated as a patch, lotion, gel, microneedle array, intralesional injection, etc. The ENAC blocker can be formulated in combination with other agents effective in the treatment of psoriasis, e.g. corticosteroids, vitamin D analogs, retinoids, and the like. Without limitation as to the mode of action, it is believed that ENAC blockers have a specificity of action on abnormal suprabasilar epidermal proliferation, and thus can provide a complementary mode of action, e.g. to agents that act on immune function and the like. The ENAC blocker can be administered in combination with systemic agents as well, e.g. immunosuppressants, anti-cytokine therapies, T-cell-targeted therapies, and the like.

Disclosed herein are methods and compositions for treating psoriasis, e.g., chronic psoriasis, using an epithelial ion channel (ENAC) blocker. The flare of psoriasis may be indicated by loss of a Psoriasis Area and Severity Index (PASI) 90 response, by loss of a Psoriasis Area and Severity Index (PASI) 75 response, by loss of a Psoriasis Area and Severity Index (PASI) 50 response, or by loss of a clear or minimal Physician's Global Assessment (PGA) rating. The loss of a PASI response may be loss of PASI response of a single body region, loss of PASI response of two body regions, loss of PASI response of three body regions, or loss of PASI response of four body regions. The body region may be trunk, lower extremities, upper extremities, or head and neck.

In one disclosure, the psoriasis is chronic psoriasis. In one disclosure, the psoriasis is plaque psoriasis, e.g., chronic plaque psoriasis. In another disclosure, the psoriasis is chronic psoriasis, e.g., chronic plaque psoriasis. In yet another disclosure, the psoriasis is moderate to severe psoriasis, e.g., moderate to severe plaque psoriasis, moderate to severe chronic psoriasis or moderate to severe chronic plaque psoriasis. In one disclosure, the subject has had a clinical diagnosis of psoriasis for at least 6 months. In another disclosure, the subject has had stable plaque psoriasis for at least 2 months.

### BRIEF DESCRIPTION OF THE DRAWINGS

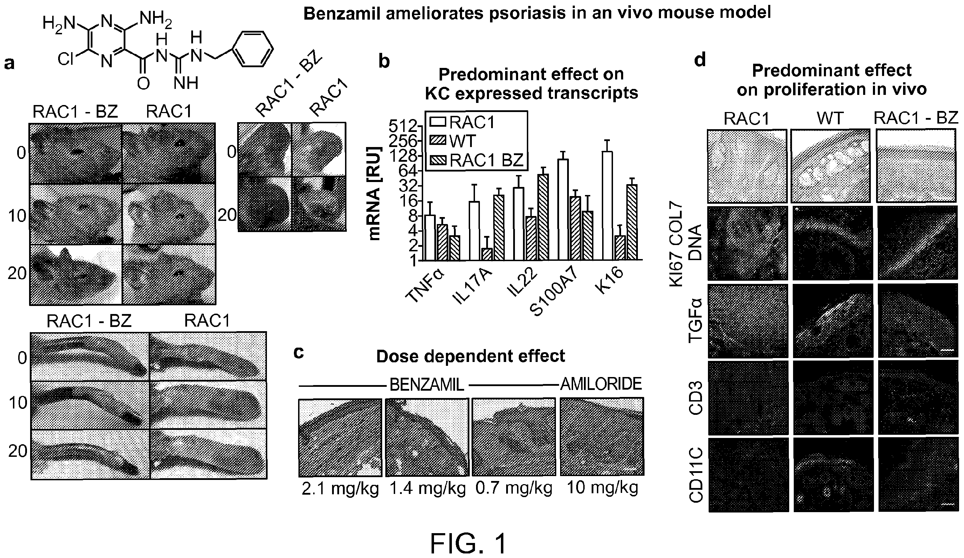

FIGURES 1A-1D: IP treatment with benzamil ameliorates phenotype in a mouse model of psoriasis. (a) 20 intraperitoneal injections of 2mg/kg Benzamil every other day reversed erythema, scaling and swelling, including on muzzle, tail and ears. (b) Keratinocyte-derived mRNA transcripts (K16, S100A7) were predominately affected in treated skin compared to mRNA transcripts mainly expressed by immune cells (IL17, IL22). (c) IP efficacy was dose-dependent (2.1-1.4-0.7 mg/kg 20 treatments) and distinct from another ENAC blocker Amiloride (10mg/kg). (d) Histologic analysis demonstrated marked reduction of psoriasiform hyperplasia in treated mice, and reduced TGFa and suprabasal proliferation (ki67). No differences were found in T- (CD3+) or dendritic (cd11c+) cell counts in treated compared to untreated skin.

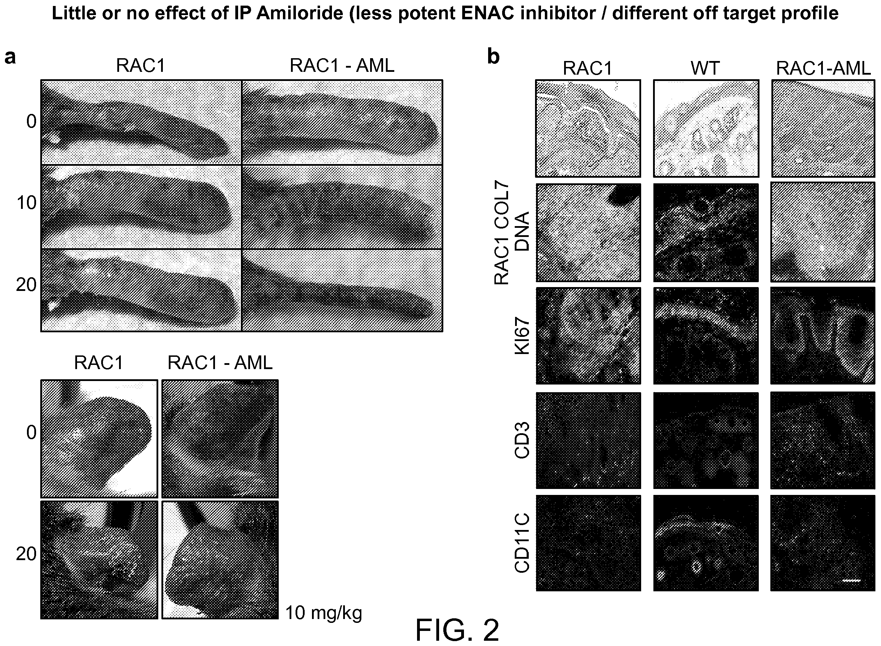

FIGURES 2A-2B: Little or no effect of IP Amiloride (less potent ENAC inhibitor/ different offtarget profile IP treatment with amiloride (10mg/kg every other day 20 treatments) reduced edema but did not have distinct effect on scaling (a). Histologically, retained psoriasiform hyperplasia was evident, with high Rac1 activation, ki67 and TGFa expression and immune cell infiltration (CD3+/cd11c+) (b).

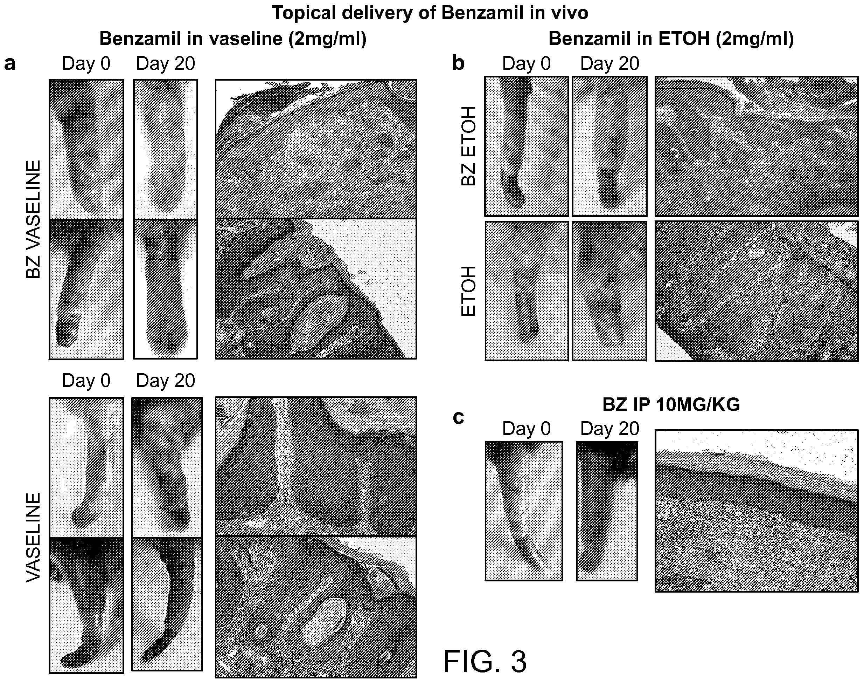

FIGURES 3A-3C: Topical delivery of Benzamil in vivo. 20 days of topical once daily treatment with benzamil in Vaseline (2mg/ml) (a) or benzamil in 70% ETOH (2mg/ml) (b) reduced scaling, erythema and epidermal thickening. Comparison with ip administration (c).

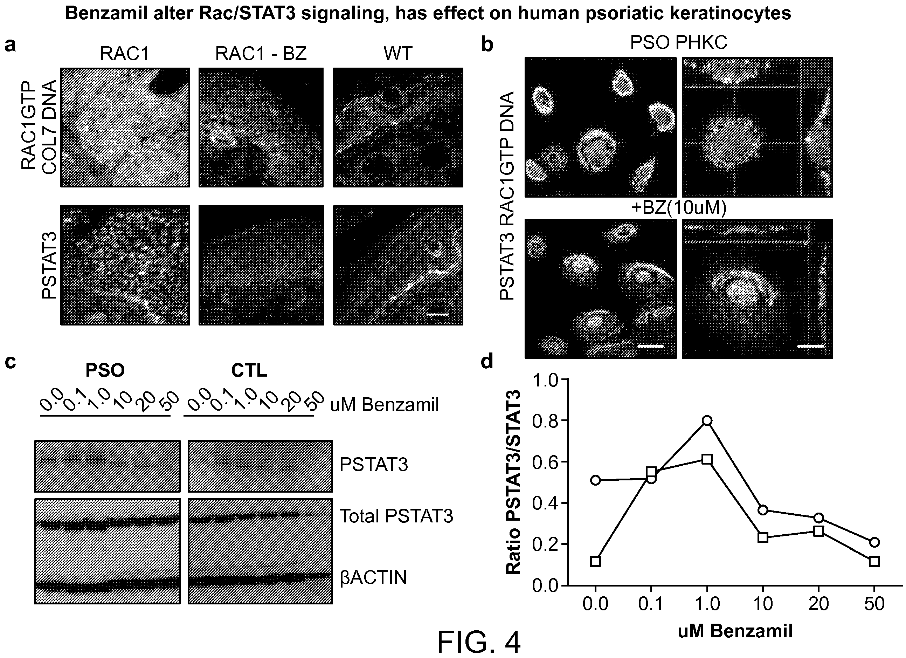

FIGURES 4A-4D: Benzamil alter Rac/STAT3 signaling, has effect on human psoriatic keratinocytes. IP treatment with benzamil (2mg/kg) markedly reduced and relocalized Rac1GTP in treated lesional skin, and reduced epidermal phosphorylation of STAT3 (a). Effects on Rac1 and PSTAT3 by Benzamil (10uM) were validated in human primary psoriatic keratinocytes (Rac1-green, PSTAT3-red, DNA-blue) (b). Western blots of cell lysates from primary psoriatic or control keratinocytes showed a dose-dependent reduction in phosphorylated STAT3 (c), ratio PSTAT3/total STAT3 quantified in (d).

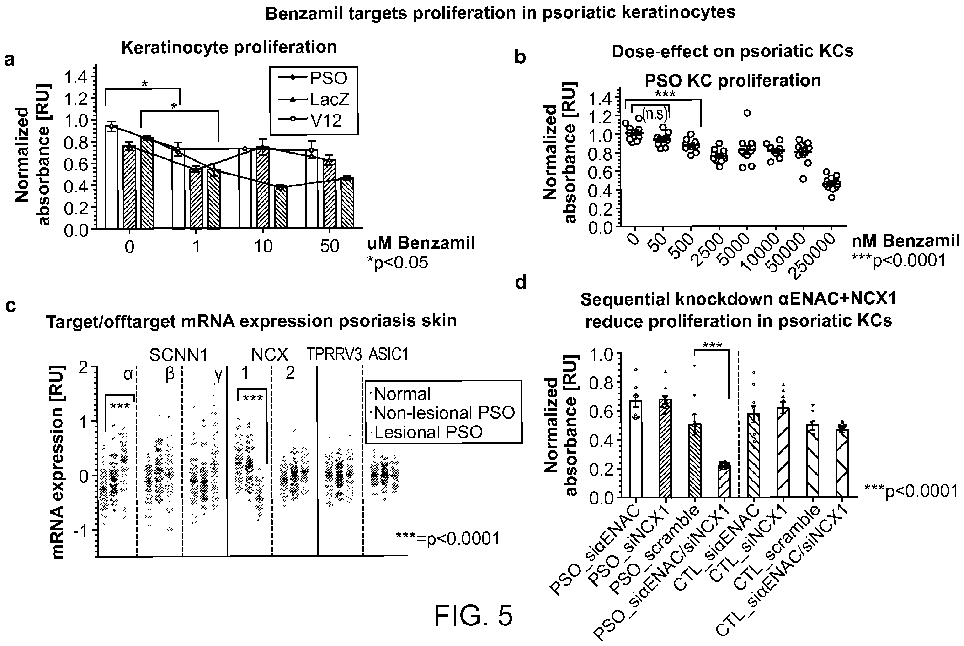

FIGURES 5A-5D: Benzamil targets proliferation in psoriatic keratinocytes. MTT assay of proliferation in V12-transduced primary normal keratinocytes compared to primary (LacZ) psoriatic keratinocytes or (LacZ) control primary keratinocytes showed a dose-dependent reduction in proliferation by Benzamil (a). Significant decrease in psoriatic keratinocytes proliferation was evident at 500 nM Benzamil concentrations (b). Comparison of mRNA expression of benzamil targets in a panel of human psoriasis lesional and non-lesional biopsies compared to control skin identified significant differences in both aENAC and NCX1 (c). MTT assay of siRNA transduced aENAC, NCX or scrambled psoriatic and control keratinocytes, respectively, identified significantly reduced proliferation upon sequential knockdown of both aENAC and NCX1 (d).

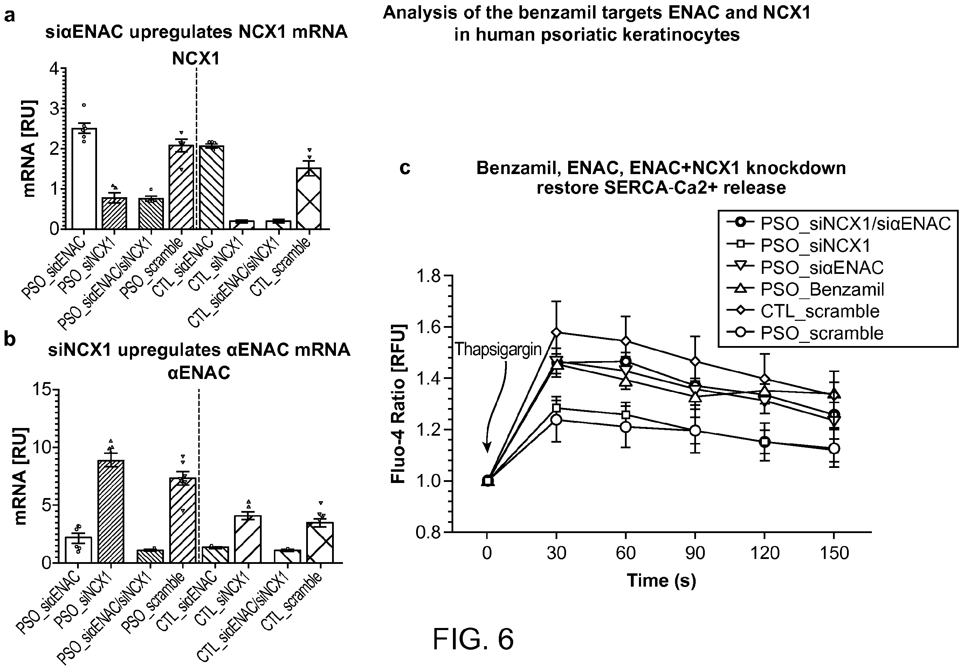

FIGURES 6A-6C: Analysis of the benzamil targets ENAC and NCX1 in human psoriatic keratinocytes. siRNA knockdown of aENAC upregulated NCX1 expression (a) and siRNA knockdown of NCX1 upregulated aENAC expression (b). Benzamil restored the intrinsic Ca²⁺ buffering capacity of psoriatic keratinocytes, which was mimicked by sequential knockdown of both aENAC and NCX1 (c).

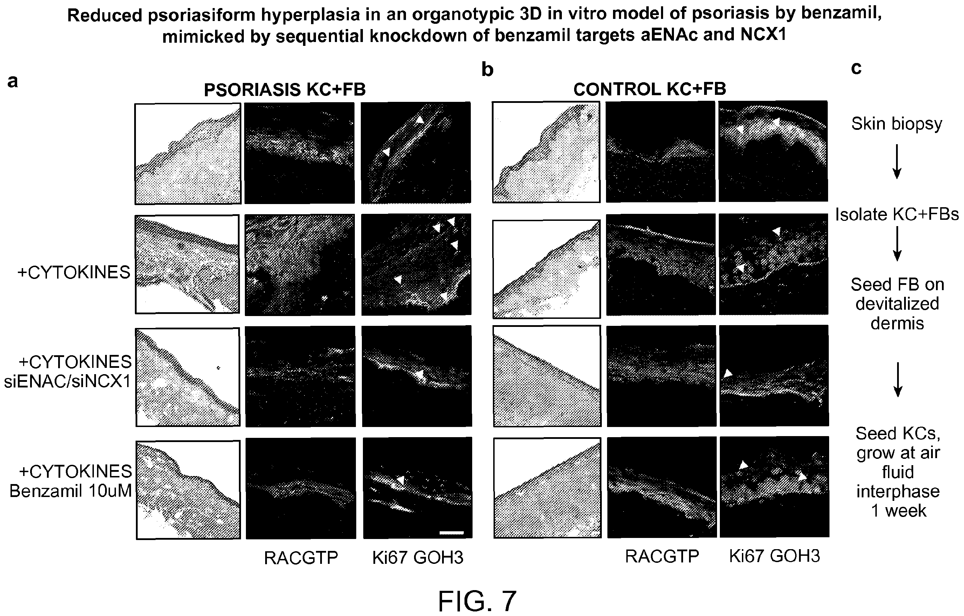

FIGURES 7A-7C: Reduced psoriasiform hyperplasia in an organotypic 3D in vitro model of psoriasis by benzamil, mimicked by sequential knockdown of benzamil targets aENAc and NCX1. Through isolation of primary keratinocytes and autologous fibroblasts, seeded on devitalized dermis and grown in air-fluid interphase, cytokine stimulation (IL23 25ng/ml;, IL17A/F 100ng/ml; TNFa 100ng/ml and IL22 25ng/ml) induced psoriasform hyperplasia, Rac1GTP expression and basal and suprabasal proliferation (ki67) in psoriasis cells, which was rescued by either Benzamil treatment (10uM) or sequential knockdown of aENAC and NCX1 (a). In contrast, conditions with control cells did not induce psoriasiform hyperplasia upon cytokine stimulation. Workflow depicted in (c).

### DETAILED DESCRIPTION OF THE EMBODIMENTS

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. In this specification and the appended claims, the singular forms "a," "an" and "the" include plural reference unless the context clearly dictates otherwise.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, illustrative methods, devices and materials are now described.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims.

*Epithelial ion channel (ENAC) blocker.* ENAC blockers are compounds, typically small molecules, that directly block the epithelial sodium channel (ENaC), thereby inhibiting sodium reabsorption. Known blockers include triamterene, phenamil, amiloride and amiloride derivatives, particularly benzyl amiloride (Benzamil). Additional amiloride derivatives are described in WO2012035158; WO2009074575; WO2011028740; WO2009150137; WO2011079087; and WO2008135557. Benzamil is used in the methods and compositions of the invention.

*Chronic Plaque Psoriasis.* Chronic plaque psoriasis (also referred to as psoriasis vulgaris) is the most common form of psoriasis. Chronic plaque psoriasis is characterized by raised reddened patches of skin, ranging from coin-sized to much larger. In chronic plaque psoriasis, the plaques may be single or multiple, they may vary in size from a few millimeters to several centimeters. The plaques are usually red with a scaly surface, and reflect light when gently scratched, creating a "silvery" effect. Lesions (which are often symmetrical) from chronic plaque psoriasis occur all over body, but with predilection for extensor surfaces, including the knees, elbows, lumbosacral regions, scalp, and nails. Occasionally chronic plaque psoriasis can occur on the penis, vulva and flexures, but scaling is usually absent. Diagnosis of patients with chronic plaque psoriasis is usually based on the clinical features described above. In particular, the distribution, color and typical silvery scaling of the lesion in chronic plaque psoriasis are characteristic of chronic plaque psoriasis.

*Guttate Psoriasis.* Guttate psoriasis refers to a form of psoriasis with characteristic water drop shaped scaly plaques. Flares of guttate psoriasis generally follow an infection, most notably a streptococcal throat infection. Diagnosis of guttate psoriasis is usually based on the appearance of the skin, and the fact that there is often a history of recent sore throat.

*Inverse Psoriasis.* Inverse psoriasis is a form of psoriasis in which the patient has smooth, usually moist areas of skin that are red and inflamed, which is unlike the scaling associated with plaque psoriasis. Inverse psoriasis is also referred to as intertiginous psoriasis or flexural psoriasis. Inverse psoriasis occurs mostly in the armpits, groin, under the breasts and in other skin folds around the genitals and buttocks, and, as a result of the locations of presentation, rubbing and sweating can irritate the affected areas.

*Pustular Psoriasis.* Pustular psoriasis, also referred to as palmar plantar psoriasis, is a form of psoriasis that causes pus-filled blisters that vary in size and location, but often occur on the hands and feet. The blisters may be localized, or spread over large areas of the body. Pustular psoriasis can be both tender and painful, can cause fevers.

*Erythrodermic Psoriasis.* Erythrodermic psoriasis is a particularly inflammatory form of psoriasis that often affects most of the body surface. It may occur in association with von Zumbusch pustular psoriasis. It is a rare type of psoriasis, occurring once or more during the lifetime of 3 percent of people who have psoriasis. It generally appears on people who have unstable plaque psoriasis. Widespread, fiery redness and exfoliation of the skin characterize this form. Severe itching and pain often accompanies it. Erythrodermic psoriasis causes protein and fluid loss that can lead to severe illness. Edema (swelling from fluid retention), especially around the ankles, may develop, along with infection. Erythrodermic psoriasis also can bring on pneumonia and congestive heart failure. People with severe cases often require hospitalization. Erythrodermic psoriasis can occur abruptly at the first signs of psoriasis or it can come on gradually in people with plaque psoriasis. Combination treatments are frequently required, for example topical products and one or two systemic medications.

The term "sensitivity" and "sensitive" when made in reference to treatment is a relative term which refers to the degree of effectiveness of a treatment compound in lessening or decreasing the symptoms of the disease being treated. For example, the term "increased sensitivity" when used in reference to treatment of a cell or patient refers to an increase of, at least a 5%, or more, in the effectiveness in lessening or decreasing the symptoms of psoriasis when measured using any methods well-accepted in the art.

As used herein, and unless otherwise specified, the term "therapeutically effective amount" of a compound is an amount sufficient to provide a therapeutic benefit in the treatment or management of psoriasis, or to delay or minimize one or more symptoms associated with psoriasis. A therapeutically effective amount of a compound means an amount of therapeutic agent, alone or in combination with other therapies, which provides a therapeutic benefit in the treatment or management of psoriasis. The term "therapeutically effective amount" can encompass an amount that improves overall therapy, reduces or avoids symptoms or causes of psoriasis, or enhances the therapeutic efficacy of another therapeutic agent.

The term "likelihood" generally refers to an increase in the probability of an event. The term "likelihood" when used in reference to the effectiveness of a patient response generally contemplates an increased probability that the symptoms of psoriasis will be lessened or decreased.

The terms "determining", "measuring", "evaluating", "assessing" and "assaying" as used herein generally refer to any form of measurement, and include determining if an element is present or not. These terms include both quantitative and/or qualitative determinations. Assessing may be relative or absolute. "Assessing the presence of" can include determining the amount of something present, as well as determining whether it is present or absent.

The term "sample" as used herein relates to a material or mixture of materials, typically, although not necessarily, in fluid form, containing one or more components of interest.

"Biological sample" as used herein refers to a sample obtained from a biological subject, including sample of biological tissue or fluid origin, obtained, reached, or collected in vivo or in situ. A biological sample also includes samples from a region of a biological subject containing precancerous or cancer cells or tissues. Such samples can be, but are not limited to, organs, tissues, fractions and cells isolated from a mammal. Exemplary biological samples include but are not limited to cell lysate, a cell culture, a cell line, a tissue, oral tissue, gastrointestinal tissue, an organ, an organelle, a biological fluid, a blood sample, a urine sample, a skin sample, and the like. Preferred biological samples include but are not limited to whole blood, partially purified blood. PBMCs, tissue biopsies, and the like.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present disclosure, therefore, includes methods of combination therapeutic treatment and combination pharmaceutical compositions.

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (e.g., human).

As used herein, the term "dose amount" refers to the quantity, e.g., milligrams (mg), of the substance which is administered to the subject. In one disclosure, the dose amount is a fixed dose, e.g., is not dependent on the weight of the subject to which the substance is administered. In another disclosure, the dose amount is not a fixed dose, e.g., is dependent on the weight of the subject to which the substance is administered, or for a topical therapy a dose may be related to the surface area that is treated, e.g. dose/m² of skin.

Exemplary dose amounts, e.g., fixed dose amounts, for use treating an adult human by the methods disclosed herein include, about 0.01 mg, about 0.05 mg, about 0.1 mg, about 0.5 mg, about 1 mg, about 5 mg, about 10 mg, about 50 mg, about 100 mg, about 500 mg, or more.

Exemplary dose amounts, e.g., dose amounts for topical use treating an adult human by the methods disclosed herein include, about 0.01 mg/m² surface area, about 0.05 mg/m² surface area, about 0.1 mg/m² surface area, about 0.5 mg/m² surface area, about 1 mg/m² surface area, about 5 mg/m² surface area, about 10 mg/m² surface area, about 50 mg/m² surface area, about 100 mg/m² surface area, about 500 mg/m² surface area, or more.

Ranges intermediate to the above-recited ranges are also contemplated by the invention. For example, ranges having any one of these values as the upper or lower limits are also intended to be part of the invention, e.g., about 0.01 mg to about 100 mg, about 1 mg to about 10 mg, etc.

As used herein, the term "periodicity" as it relates to the administration of a substance refers to a (regular) recurring cycle of administering the substance to a subject. In one disclosure, the recurring cycle of administration of the substance to the subject achieves a therapeutic objective. The periodicity of administration of the substance may be about once a week, once every other week, about once every three weeks, about once every 4 weeks, about once every 5 weeks, about once every 6 weeks, about once every 7 weeks, about once every 8 weeks, about once every 9 weeks, about once every 10 weeks, about once every 11 weeks, about once every 12 weeks, about once every 13 weeks, about once every 14 weeks, about once every 15 weeks, about once every 16 weeks, about once every 17 weeks, about once every 18 weeks, about once every 19 weeks, about once every 20 weeks, about once every 21 weeks, about once every 22 weeks, about once every 23 weeks, about once every 24 weeks, about once every 5-10 days, about once every 10-20 days, about once every 10-50 days, about once every 10-100 days, about once every 10-200 days, about once every 25-35 days, about once every 20-50 days, about once every 20-100 days, about once every 20-200 days, about once every 30-50 days, about once every 30-90 days, about once every 30-100 days, about once every 30-200 days, about once every 50-150 days, about once every 50-200 days, about once every 60-180 days, or about once every 80-100 days. Periodicities intermediate to the above-recited times are also disclosed Ranges intermediate to the above-recited ranges are also disclosed. For example, ranges having any one of these values as the upper or lower limits are also disclosed, e.g., about 110 days to about 170 days, about 160 days to about 220 days, etc.

The "duration of a periodicity" refers to a time over which the recurring cycle of administration occurs. For example, a duration of the periodicity of administration of a substance may be may be up to about 4 weeks, up to about 8 weeks, up to about 12 weeks, up to about 16 weeks or more, up to about 20 weeks, up to about 24 weeks, up to about 28 week, up to about 32 weeks or more, during which the periodicity of administration is about once every week. For example, a duration of the periodicity may be about 6 weeks during which the periodicity of administration is about once every 4 weeks, e.g., the substance is administered at week zero and at week four.

In one disclosure, the duration of periodicity is for a length of time necessary or required to achieve a therapeutic objective, e.g., treatment, maintenance of treatment, etc. e.g., maintain a PASI 50, PASI 75, PASI 90, PASI 100 score or PGA of 0 or 1 score. Durations of a periodicity intermediate to the above-recited times are also disclosed.

As used herein, and unless otherwise specified, the terms "treat," "treating" and "treatment" refer to an action that occurs while a patient is suffering from psoriasis, which reduces the severity of psoriasis, or retards or slows the progression of the psoriasis, or achieving or maintaining a therapeutic objective. An "effective patient response" refers to any increase in the therapeutic benefit to the patient. An "effective patient psoriasis response" can be, for example, a 5%, 10%, 25%, 50%, or 100% decrease in the physical symptoms of psoriasis.

"Treatment of or "treating" psoriasis may mean achieving or maintaining a PGA score of 0/1 or a PASI 50, PASI 75, PASI 90, or PASI 100 response score for a period of time during or following treatment (e.g., for at least 2, 4, 6, 8, 10, 12, 14, 16, 18, 20, 22, 24, 26, 28, 30, 32, 34, 36, 38, 40, 42, 46, 48, 50, 52, 54, 56, 58 or 60 weeks or longer). "Treatment of or "treating" psoriasis may also mean achieving or maintaining a health-related quality of life (HRQOL) outcome. HRQOL outcomes include Dermatology Life Quality Index (DLQI), visual analog scales for Ps-related (VAS-Ps) and psoriatic arthritis-related (VAS-PsA) pain, Short Form 36 Health Survey Mental (MCS) and Physical (PCS) Component Summary scores, and Total Activity Impairment (TAI) scores.

"Treatment of or "treating" psoriasis may also mean achieving or maintaining a minimum clinically important difference (MCID) for any of the HRQOL outcomes provided herein, e.g., any one or combination of DLQI, VAS-Ps, VAS-PsA, MCS, PCS and TAI.

"Treatment of" or "treating" psoriasis may also mean achieving or maintaining a minimum clinically important difference (MCID) response rate for any of the HRQOL outcomes provided herein, e.g., any one or combination of DLQI, VAS-Ps, VAS-PsA, MCS, PCS and TAI. "Treatment of or "treating" psoriasis may also mean achieving or maintaining a clinically meaningful reduction in any of the HRQOL outcomes provided herein, e.g., any one or combination of DLQI, VAS-Ps, VAS-PsA, MCS, PCS and TAI.

"Treatment of or "treating" psoriasis may also mean achieving or maintaining a Nail Psoriasis Severity Index (NAPSI) score for a period of time during or following treatment (e.g., for at least 2, 4, 6, 8, 10, 12, 14, 16, 18, 20, 22, 24, 26, 28, 30, 32, 34, 36, 38, 40, 42, 46, 48, 50, 52, 54, 56, 58 or 60 weeks or longer).

"Treatment of" or "treating" psoriasis may also mean achieving or maintaining any of the outcomes provided herein in a certain percentage of a population of subjects (e.g., in at least about 10%, 15%, 20%, 25%, 30%, 35%, 40%, 45%, 50%, 55%, 60%, 65%, 70%, 75%, 80%, 85%, 90%, 95%, 99%, or 100% of a population of subjects).

The term "kit" as used herein refers to a packaged product comprising components with which to administer the epithelial ion channel blocker disclosed herein for treatment of psoriasis. The kit preferably comprises a box or container that holds the components of the kit. The box or container may be affixed with a label or a Food and Drug Administration approved protocol. The box or container holds components disclosed herein which are preferably contained within plastic, polyethylene, polypropylene, ethylene, or propylene vessels. The vessels can be capped-tubes or bottles. The kit can also include instructions for use.

Formulations. Disclosed herein are pharmaceutically acceptable compositions which comprise a therapeutically-effective amount of at least one ENAC blocker, e.g. Benzamil, optionally combined with one or more additional agents for treatment of psoriasis, formulated together with one or more pharmaceutically acceptable excipients. The active ingredients and excipient(s) may be formulated into compositions and dosage forms according to methods known in the art. As described in detail below, the pharmaceutical compositions disclosed herein may be specially formulated for administration in solid or liquid form, including those adapted for the following: oral administration, for example, tablets, capsules, powders, granules, pastes for application to the tongue, aqueous or non-aqueous solutions or suspensions, drenches, or syrups; parenteral administration, for example, by subcutaneous, intramuscular or intravenous injection as, for example, a sterile solution or suspension. In other disclosures the formulation is provided for topical application, for example, as a lotion, cream, ointment, spray, patch, microneedle array, *etc.* applied to the skin.

The phrase "pharmaceutically acceptable" is employed herein to refer to those compounds, materials, compositions, and/or dosage forms which are, within the scope of sound medical judgment, suitable for use in contact with the tissues of the subject with toxicity, irritation, allergic response, or other problems or complications, commensurate with a reasonable benefit/risk ratio.

The phrase "pharmaceutically-acceptable excipient" as used herein refers to a pharmaceutically-acceptable material, composition or vehicle, such as a liquid or solid filler, diluent, carrier, manufacturing aid (e.g., lubricant, talc magnesium, calcium or zinc stearate, or steric acid), solvent or encapsulating material, involved in carrying or transporting the therapeutic compound for administration to the subject. Each excipient should be "acceptable" in the sense of being compatible with the other ingredients of the formulation and not injurious to the subject. Some examples of materials which can serve as pharmaceutically-acceptable excipients include: ethanol, sugars, such as lactose, glucose and sucrose; starches, such as corn starch and potato starch; cellulose and its derivatives, such as sodium carboxymethyl cellulose, ethyl cellulose and cellulose acetate; gelatin; talc; waxes; oils, such as peanut oil, cottonseed oil, safflower oil, sesame oil, olive oil, corn oil and soybean oil; glycols, such as ethylene glycol and propylene glycol; polyols, such as glycerin, sorbitol, mannitol and polyethylene glycol; esters, such as ethyl oleate and ethyl laurate; agar; buffering agents; water; isotonic saline; pH buffered solutions; and other non-toxic compatible substances employed in pharmaceutical formulations. If desired, certain sweetening and/or flavoring and/or coloring agents may be added. Other suitable excipients can be found in standard pharmaceutical texts, e.g. in "Remington's Pharmaceutical Sciences", The Science and Practice of Pharmacy, 19.sup.th Ed. Mack Publishing Company, Easton, Pa., (1995).

Excipients are added to the composition for a variety of purposes. Diluents increase the bulk of a solid pharmaceutical composition, and may make a pharmaceutical dosage form containing the composition easier for the patient and caregiver to handle. Diluents for solid compositions include, for example, microcrystalline cellulose, microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit), potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose, hydroxypropyl methyl cellulose, liquid glucose, magnesium aluminum silicate, maltodextrin, methylcellulose, polymethacrylates, povidone, pregelatinized starch, sodium alginate and starch. The dissolution rate of a compacted solid pharmaceutical composition in the subjects's stomach may be increased by the addition of a disintegrant to the composition. Disintegrants include alginic acid, carboxymethylcellulose calcium, carboxymethylcellulose sodium, colloidal silicon dioxide, croscarmellose sodium, crospovidone, guar gum, magnesium aluminum silicate, methyl cellulose, microcrystalline cellulose, polacrilin potassium, powdered cellulose, pregelatinized starch, sodium alginate, sodium starch glycolate and starch.

In liquid pharmaceutical compositions disclosed herein, the agent and any other solid excipients are dissolved or suspended in a liquid carrier such as water, water-for-injection, vegetable oil, alcohol, polyethylene glycol, propylene glycol or glycerin. Liquid pharmaceutical compositions may contain emulsifying agents to disperse uniformly throughout the composition an active ingredient or other excipient that is not soluble in the liquid carrier. Emulsifying agents that may be useful in liquid compositions disclosed herein include, for example, gelatin, egg yolk, casein, cholesterol, acacia, tragacanth, chondrus, pectin, methyl cellulose, carbomer, cetostearyl alcohol and cetyl alcohol. Liquid pharmaceutical compositions disclosed herein may also contain a viscosity enhancing agent to improve the mouth-feel of the product and/or coat the lining of the gastrointestinal tract. Sweetening agents such as sorbitol, saccharin, sodium saccharin, sucrose, aspartame, fructose, mannitol and invert sugar may be added to improve the taste. Flavoring agents and flavor enhancers may make the dosage form more palatable to the patient. Preservatives and chelating agents such as alcohol, sodium benzoate, butylated hydroxy toluene, butylated hydroxyanisole and ethylenediamine tetraacetic acid may be added at levels safe for ingestion to improve storage stability. Selection of excipients and the amounts used may be readily determined by the formulation scientist based upon experience and consideration of standard procedures and reference works in the field.

In several disclosures herein the ENAC blocker, e.g. Benzamil, or formulated for topical application to the skin Various specific formulations are provided, including lotions, gels, liquids, patches, intralesional injection, and the like. A typical dose for a topical formulation in lotion or liquid form is from about 1 µl to about 100 µl to about 1 ml, to about 10 ml, applied in a lotion, cream, gel, etc. to the affected skin.

In general, the subject formulations will typically contain at least about 1 µg/ml active agent, at least about 10 µg/ml, at least about 50 µg/ml, at least about 100 µg/ml, at least about 500 µg/ml, and not more than about 100 mg/ml. In some disclosures the formulation comprises at least about 0.1 mM, at least about 0.05, at least about 1 mM, at least about 5 mM, at least about 10 mM, at least about 50 mM. The active agents of the present disclosure are formulated at an effective concentration within the subject formulations, meaning at a concentration that provides the intended benefit when applied topically.

The dose of active agent is as described above with respect to the surface area to be treated, where the dose may be up to about 0.01 mg/kg body weight, up to about 0.05 mg/kg body weight, up to about 0.1 mg/kg body weight, up to about 0.5 mg/kg body weight, up to about 1 mg/kg body weight, up to about 2 mg/kg body weight, up to about 5 mg/kg body weight, up to about 10 mg/kg body weight.

Administration may be every 6 hours, every 12 hours, every 24 hours, every 48 hours, every 3 days, every 4 days, every 5 days, weekly, biweekly, monthly, etc. In various of these disclosures, the therapeutically effective dose is administered on consecutive days for at least a week, at least a month, at least a year, or on as needed basis for the rest of the patient's life. The therapeutically effective dose, e.g. of Benzamil, or pharmaceutically acceptable salt thereof, can be about 10-500 mg/day, about 50-400 mg/day, about 100-200 mg/day, or about 120-180 mg/day. Benzamil or pharmaceutically acceptable salt thereof, can be administered to a subject at about 1-110 mg daily, 1-100 mg twice a day, 1-100 mg. every other day, as needed.

Examples are provided herein of dosages useful for treatment of an animal model. As is known in the art, in order to convert dosage from, for example, a mouse to a human, the animal dose should not be extrapolated to a human equivalent dose (HED) by a simple conversion based on body weight. The more appropriate conversion of drug doses from animal studies to human studies, uses the body surface area (BSA) normalization method. BSA correlates well across several mammalian species with several parameters of biology, including oxygen utilization, caloric expenditure, basal metabolism, blood volume, circulating plasma proteins, and renal function. See, for example, Reagan-Shaw et al. (2008) The FASEB Journal 22(3), 659-661. The appropriate dose for a human may be roughly 1/10^{th} to 1/20^{th} of the dose for a mouse. See also, FDA guidance for Estimating the Maximum Safe Starting Dose in Initial Clinical Trials for Therapeutics in Adult Healthy Volunteers.

In some disclosures, the topical formulation comprises skin penetration enhancers. Such enhancers reversibly decrease skin barrier resistance, and include without limitation, sulphoxides (such as dimethylsulphoxide, DMSO), azones (e.g. laurocapram), pyrrolidones (for example 2-pyrrolidone, 2P), alcohols and alkanols (ethanol, or decanol), glycols (for example propylene glycol, PG, a common excipient in topically applied dosage forms), surfactants (also common in dosage forms) and terpenes.

Topical formulations include lotions, gels, creams, etc. Such formulations may include a pharmaceutically acceptable vehicle to act as a dilutant, dispersant or carrier for the active agent(s), so as to facilitate distribution when the composition is applied to the skin. Vehicles other than or in addition to water can include liquid or solid emollients, solvents, humectants, thickeners and powders. The vehicle will usually form from 5% to 99.9%, preferably from 25% to 80% by weight of the composition, and can, in the absence of other cosmetic adjuncts, form the balance of the composition. The compositions may be in the form of aqueous, aqueous/alcoholic or oily solutions; dispersions of the lotion or serum type; anhydrous or lipophilic gels; emulsions of liquid or semi-liquid consistency, which are obtained by dispersion of a fatty phase in an aqueous phase (O/W) or conversely (W/O); or suspensions or emulsions of smooth, semi-solid or solid consistency of the cream or gel type. These compositions are formulated according to the usual techniques as are well known to this art.

When formulated as an emulsion, the proportion of the fatty phase may range from 5% to 80% by weight, and preferably from 5% to 50% by weight, relative to the total weight of the composition. Oils, emulsifiers and co-emulsifiers incorporated in the composition in emulsion form are selected from among those used conventionally in the cosmetic or dermatological field. The emulsifer and coemulsifier may be present in the composition at a proportion ranging from 0.3% to 30% by weight, and preferably from 0.5% to 20% by weight, relative to the total weight of the composition. When the lotions are formulated as an oily solution or gel, the fatty phase may constitute more than 90% of the total weight of the composition.

Formulations may also contain additives and adjuvants which are conventional in the cosmetic, pharmaceutical or dermatological field, such as hydrophilic or lipophilic gelling agents, hydrophilic or lipophilic active agents, preservatives, antioxidants, solvents, fragrances, fillers, bactericides, odor absorbers and dyestuffs or colorants. The amounts of these various additives and adjuvants are those conventionally used in the field, and, for example, range from 0.01% to 10% of the total weight of the composition. Depending on their nature, these additives and adjuvants may be introduced into the fatty phase, into the aqueous phase.

Exemplary oils which may be used according to this disclosure include mineral oils (liquid petrolatum) and solid oils, e.g. petrolatum, plant oils (liquid fraction of karite butter, sunflower oil), animal oils (perhydrosqualen(e), synthetic oils (purcellin oil), silicone oils (cyclomethicone) and fluoro oils (perfluoropolyethers). Fatty alcohols, fatty acids (stearic acid) and waxes (paraffin wax, carnauba wax and beeswax) may also be used as fats. Emulsifiers which may be used include glyceryl stearate, polysorbate 60, PEG-6/PEG-32/glycol stearate mixture, *etc.* Solvents which may be used include the lower alcohols, in particular ethanol and isopropanol, and propylene glycol. Hydrophilic gelling agents include carboxyvinyl polymers (carbomer), acrylic copolymers such as acrylate/alkylacrylate copolymers, polyacrylamides, polysaccharides, such as hydroxypropylcellulose, natural gums and clays, and, as lipophilic gelling agents, representative are the modified clays such as bentones, fatty acid metal salts such as aluminum stearates and hydrophobic silica, or ethylcellulose and polyethylene.

An oil or oily material may be present, together with an emollient to provide either a water-in- oil emulsion or an oil-in-water emulsion, depending largely on the average hydrophilic-lipophilic balance (HLB) of the emollient employed. Levels of such emollients may range from about 0.5% to about 50%, preferably between about 5% and 30% by weight of the total composition. Emollients may be classified under such general chemical categories as esters, fatty acids and alcohols, polyols and hydrocarbons. Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids. Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients for lotions are thickeners. A thickener will usually be present in amounts anywhere from 0.1 to 20% by weight, preferably from about 0.5% to 10% by weight of the composition. Exemplary thickeners are cross-linked polyacrylate materials available under the trademark Carbopol. Gums may be employed such as xanthan, carrageenan, gelatin, karaya, pectin and locust beans gum. Under certain circumstances the thickening function may be accomplished by a material also serving as a silicone or emollient. For instance, silicone gums in excess of 10 centistokes and esters such as glycerol stearate have dual functionality. Powders may be incorporated into a lotion. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

An alternative formulation for topical delivery is an array of microneedles. Microneedles (MN), as used herein, refers to an array comprising a plurality of micro-projections, generally ranging from about 25 to about 2000 µm in length, which are attached to a base support. An array may comprise 10², 10³, 10⁴, 10⁵ or more microneedles, and may range in area from about 0.1 cm² to about 100 cm². Application of MN arrays to biological membranes creates transport pathways of micron dimensions, which readily permit transport of macromolecules such as large polypeptides. In some disclosures herein, the microneedle array is formulated as a transdermal drug delivery patch. MN arrays can alternatively be integrated within an applicator device which, upon activation, can deliver the MN array into the skin surface, or the MN arrays can be applied to the skin and the device then activated to push the MN through the SC.

Various materials have been used for microneedles. For example, biodegradable materials into which the therapeutic agent, e.g. Benzamil, can be incorporated are of interest. Such materials include various biodegradable or biocompatible polymers or cross-linked monomers, as known in the art. The dose of agent to be delivered will vary, and may range from at least about 1 ng/microneedle array, at least about 10 ng, at least about 0.1 µg, at least about 1 µg, at least about 10 µg, at least 0.1 mg, at least 1 mg, or more in a single array. MNs may be fabricated with a wide range of designs (different sizes and shapes) and different types (solid, hollow, sharp, or flat), and may be in-plane and/or out-of-plane.

Polymeric MNs can provide biocompatibility, biodegradability, strength, toughness, and optical clarity. To accurately produce the micro-scale dimensions of polymer MNs, a variety of mould-based techniques, such as casting, hot embossing, injection molding, and investment molding may be used, e.g. beveled-tip, chisel-tip, and tapered-cone polydimethylsiloxane (PDMS) molds. Polymeric materials of interest for fabrication include without limitation; poly (methylmetha-acrylate) (PMMA), poly-L-lactic acid (PLA), poly-glycolic acid (PGA), and poly-lactic-co-glycolic acid (PLGA), cyclic-olefin copolymer, poly (vinyl pyrrolidone), and sodium carboxymethyl cellulose. Sugars have also been used to fabricate the MNs, such as galactose, maltose, aliginate, chitosan, and dextrin. Materials may be cross-linked through ion exchange, photo-polymerization, and the like.

In other disclosures, a topical formulation is provided as a transdermal patch. Medical dressings suitable for formulation in a transdermal patch can be any material that is biologically acceptable and suitable for placing over the skin. In exemplary disdosures, the support may be a woven or non-woven fabric of synthetic or non-synthetic fibers, or any combination thereof. The dressing may also comprise a support, such as a polymer foam, a natural or man-made sponge, a gel or a membrane that may absorb or have disposed thereon, a therapeutic composition. A gel suitable for use as a support is sodium carboxymethylcellulose 7H 4F, i.e. ethylcellulose.

For example, hydrocolloids (eg, RepliCare, DuoDERM, Restore, Tegasorb), which are combinations of gelatin, pectin, and carboxymethylcellulose in the form of wafers, powders, and pastes; some have adhesive backings and others are typically covered with transparent films to ensure adherence. Alginates (polysaccharide seaweed derivatives containing alginic acid), which come as pads, ropes, and ribbons (AlgiSite, Sorbsan, Curasorb), are indicated for extensive exudate and for control of bleeding after surgical debridement. Foam dressings (Allevyn, LYOfoam, Hydrasorb, Mepilex, Curafoam, Contreet) are useful as they can handle a variety of levels of exudate and provide a moist environment for healing. Those with adhesive backings stay in place longer and need less frequent changing.

In some disclosures, a transdermal patch comprises permeation enhancer, e.g. transcutol, (diethylene glycol monoethyl ether), propylene glycol, dimethylsulfoxide (DMSO), menthol, 1-dodecylazepan-2-one (Azone), 2-nonyl-1,3- dioxolane (SEPA 009), sorbitan monolaurate (Span20), and dodecyl-2-dimethylaminopropanoate (DDAIP)., which may be provided at a weight/weight concentration of from about 0.1% to about 10%, usually from about 2.5% to about 7.5%, more usually about 5%.

Transdermal patches may further comprise additives to prevent crystallization. Such additives include, without limitation, one or more additives selected from octyldodecanol at a concentration of from about 1.5 to about 4% w/w of polymer; dextrin derivatives at a concentration of from about 2 to about 5% w/w of polymer; polyethylene glycol (PEG) at a concentration of from about 2 to about 5% w/w of polymer; polypropylene glycol (PPG) at a concentration of from about 2 to about 5% w/w of polymer; mannitol at a concentration of from about 2 to about 4% w/w of polymer; Poloxamer 407, 188, 401 and 402 at a concentration of from about 5 to about 10% w/w of polymer; and Poloxamines 904 and 908 at a concentration of from about 2 to about 6% w/w of polymer.

Polyvinylpyrrolidine (PVP) may also be included in a transdermal patch formulation, for example at a concentration of from about 5 wt% to about 25 weight%, about 7 wt% to about 20 wt%, about 8 wt% to about 18 wt%, about 10 wt% to about 16 wt%, about 10 wt%, about 12 wt%, about 14 wt%, about 16 wt%.

Emulsifiers which may be used include glyceryl stearate, polysorbate 60, PEG-6/PEG-32/glycol stearate mixture, *etc.* Solvents which may be used include the lower alcohols, in particular ethanol and isopropanol, and propylene glycol.

Hydrophilic gelling agents include carboxyvinyl polymers (carbomer), acrylic copolymers such as acrylate/alkylacrylate copolymers, polyacrylamides, polysaccharides, such as hydroxypropylcellulose, natural gums and clays, and, as lipophilic gelling agents, representative are the modified clays such as bentones, fatty acid metal salts such as aluminum stearates and hydrophobic silica, or ethylcellulose and polyethylene.

Therapeutic formulations for treatment of psoriasis with an ENAC blocker, e.g. Benzamil, can be used alone or in combination with an additional agent, e.g., a therapeutic agent, said additional agent being selected by the skilled artisan for its intended purpose. For example, the additional agent can be a therapeutic agent art-recognized as being useful to treat psoriasis. The agents set forth below are illustrative for purposes and not intended to be limited. The combinations which are disclosed herein can be an ENAC blocker and at least one additional agent selected from the lists below. The combination can also include more than one additional agent, e.g., two or three additional agents if the combination is such that the formed composition can perform its intended function.

Additional therapeutic agents include, without limitation, methotrexate, 6-MP, azathioprine sulphasalazine, mesalazine, olsalazine chloroquinine/hydroxychloroquine, pencillamine, aurothiomalate (intramuscular and oral), azathioprine, colchicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, etc., phosphodiesterase inhibitors, adensosine agonists, antithrombotic agents, complement inhibitors, adrenergic agents, agents which interfere with signaling by proinflammatory cytokines such as TNF.alpha. or IL-1 (e.g. IRAK, NIK, IKK, p38 or MAP kinase inhibitors), IL-1.beta. converting enzyme inhibitors (e.g., Vx740), anti-P7s, p-selectin glycoprotein ligand (PSGL), TNF.alpha. converting enzyme (TACE) inhibitors, T-cell signaling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors and the derivatives p75TNFRIgG and p55TNFRIgG, sIL-1RI, sIL-1RII, sIL-6R, soluble IL-13 receptor (sIL-13)) and anti-inflammatory cytokines (e.g. IL-4, IL-10, IL-11, IL-13 and TGFβ). In some disclosures the dose of the additional therapeutic agent when co-formulated with an ENAC blocker is lower than the conventional dose. In some disclosures, Benzamil is co-formulated with a glucocorticoid.

Treatment with an ENAC blocker can also be combined with PUVA therapy. PUVA is a combination of psoralen (P) and long-wave ultraviolet radiation (UVA) that is used to treat many different skin conditions. In still another disclosure, the compositions disclosed herein are administered with excimer laser treatment for treating psoriasis.

Treatment for psoriasis often includes a topical corticosteroids, vitamin D analogs, and topical or oral retinoids, or combinations thereof. In one disclosure, an ENAC blocker is administered in combination with or the presence of one of these common treatments.

The composition can be packaged in any suitable container to suit its viscosity and intended use. Disclosed herein is a closed container containing a therapeutically acceptable composition as herein defined.

The pharmaceutical compositions disclosed herein may include a "therapeutically effective amount" or a "prophylactically effective amount". A "therapeutically effective amount" refers to an amount effective, at dosages and for periods of time necessary, to achieve the desired therapeutic result. A therapeutically effective amount may vary according to factors such as the disease state, age, sex, and weight of the individual, and the ability to elicit a desired response in the individual. A therapeutically effective amount is also one in which any toxic or detrimental effects are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation.

In one disclosure, the dose is administered to the subject upon a flare of psoriasis. In another disclosure, the dose is administered to the subject prior to a flare of psoriasis.

The flare of psoriasis may be monitored by determining a subject's Psoriasis Area and Severity Index (PAST), e.g., PASI 100 response, PASI 90 response, PASI 75 response, PASI 50 response, the PASI response of a single body region, two body regions, three body regions, or four body regions, e.g., trunk, lower extremities, upper extremities, or head and neck. Alternatively, the flare of psoriasis may be monitored by determining a subject's Physician's Global Assessment (PGA) rating.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

### Methods of Use

The diagnosis of psoriasis is usually based on the appearance of the skin. Additionally a skin biopsy, or scraping and culture of skin patches may be needed to rule out other skin disorders. An x-ray may be used to check for psoriatic arthritis if joint pain is present and persistent.

A composition comprising an effective dose of an ENAC blocker, optionally combined with additional therapeutic agents, is provided to an individual with psoriasis. The administration can be oral, parenteral, topical, etc. In some disclosures topical is preferred. The dosing and periodicity of administration is selected to provide for therapeutic efficacy.

In one disclosure, the subject achieves at least a PGA score of 0 or 1. In one disclosure, the subject achieves at least a PASI 75 response. In one disclosure, the subject achieves at least a PASI 90 response. In one disclosure, the subject achieves at least a PASI 100 response. In one disclosure, the subject maintains the PGA score of 0 or 1 during treatment. In one disclosure, the subject maintains the PASI 75 response during treatment. In one disclosure, the subject maintains the PASI 90 response during treatment.

In one disclosure, the subject achieves a PGA score of 0 or 1, e.g., by about week 12. In one disclosure, the subject achieves at least a PASI 75 response, e.g., by about week 12. In one disclosure, the subject achieves at least a PASI 90 response, e.g., by about week 12. In one disclosure, the subject achieves at least a PASI 100 response, e.g., by about week 12.

In one disclosure, the subject maintains the PGA score of 0 or 1 through the duration of treatment. In one disclosure, the subject maintains the PASI 75 response through the duration of treatment. In one disclosure, the subject maintains the PASI 90 response through the duration of treatment.

In certain disclosures herein, the subject or population of subjects achieves (i) an improvement in a Dermatology Life Quality Index (DLQI) score or mean Dermatology Life Quality Index (DLQI) score of at least about -9; (ii) an improvement in a Short Form 36 Health Survey Physical Component Summary (PCS) score or mean Physical Component Summary (PCS) score of at least about 2; (iii) an improvement in a Short Form 36 Health Survey Mental Component Summary (MCS) score or mean Short Form 36 Health Survey Mental Component Summary (MCS) score of at least about 4; (iv) an improvement in a visual analog scale score or mean visual analog scale score for psoriasis-related pain (VAS-Ps) of at least about -25; (v) an improvement in a visual analog scale score for psoriatic arthritis-related pain (VAS-PsA) or mean visual analog scale score for psoriatic arthritis-related pain (VAS-PsA) of at least about -32; and/or (vi) a minimum clinically important difference (MCID) response rate for psoriasis-related pain (VAS-Ps) of at least about 60%.

Disclosed herein is a method of treating psoriasis in a population of subjects, wherein the population of subjects achieves (i) a minimum clinically important difference (MCID) response rate for Dermatology Life Quality Index (DLQI) of at least about 70% by about week 12; (ii) a minimum clinically important difference (MCID) response rate for Dermatology Life Quality Index (DLQI) of at least about 81% by about week 52; (iii) a minimum clinically important difference (MCID) response rate for Total Activity Impairment (TAI) of at least about 45% by about week 12; and/or (iv) a minimum clinically important difference (MCID) response rate for Total Activity Impairment (TAI) of at least about 57% by about week 52. In one disclosure, the antibody, or antigen-binding portion thereof, is administered once every four weeks. In another disclosure, the antibody, or antigen-binding portion thereof, is administered once every 12 weeks.

In certain disclosures herein, the subject achieves a Nail Psoriasis Severity Index (NAPSI) score of about 2.1 or less. In certain disclosures, the subject achieves a Nail Psoriasis Severity Index (NAPSI) score of about 2.1 or less by about week 24. In related disclosures herein, the subject achieves a Nail Psoriasis Severity Index (NAPSI) score of about 1.2 or less. In certain disclosures, the subject achieves a Nail Psoriasis Severity Index (NAPSI) score of about 1.2 or less by about week 52.

Animal models. In some disclosures, the compositions and methods disclosed herein are tested in an animal model, or in vitro tissue culture models, prior to treatment of a human. Psoriasis is not known to occur in animals; however, the use of animal models has provided valuable knowledge regarding the etiology of this disease. A large number of mouse models have been developed to emulate different aspects of the human condition. The first models of psoriasis were spontaneous mutations in mice which exhibited a psoriasis-like phenotype. These included mice homozygous for the asebia gene, chronic proliferative dermatitis, and the flaky skin mutations. These animals with many histological features that mimic psoriasis, however the driving mechanisms of these phenotypes appear to be independent of T cells which are known to be instrumental in this disease development.

Transgenic mice models have been employed to investigate the specific role of adhesion molecules, cytokines, transcription factors, and other mediators in the psoriasis. Epidermal overexpression of molecules of interest under the control of promoters acting in basal (e.g., keratin 14) or suprabasal keratinocytes (e.g., involucrin or keratin 10) provides information about specific epidermal functions. These latter models, however, may lack the inflammatory component of the disease. Deleting proteins within the epidermis including the inhibitor of nuclear factor- (NF-) κB-kinase 2 (IKK2), signal transducer and activator of transcription 3 (Stat3) has provided information about the role of signal transduction in psoriasiform skin inflammation.

The most widely used mouse models are xenotransplantations where a skin biopsy from a patient or produced in vitro is transplanted in mice from spontaneously mutated or genetically modified mice. The use of athymic nude mice and severe combined immunodeficient mice serve to avoid graft rejection but the former mice develop new histological changes not seen in psoriasis while the latter mice continue to manifest rejection of the xenogeneic tissue due to presence of NK cells. A new model has recently emerged where mice with spontaneous expression of AGR129 have immature NK cells and a lack of T and B cells resulting in the development of psoriatic plaques which are comparable to patient biopsies and a reduction in graft rejection. Additionally, an imiquimod-induced dermatitis mouse model has been developed which leads to psoriasis-like dermatitis.

Commonly used in vitro models of psoriasis involve the growth of human epidermal keratinocytes at an air-liquid interface resulting in the differentiation and stratification of the epidermis, hence mimicking the morphology of normal stratified squamous epidermis. The epidermal keratinocytes can be obtained from individuals with psoriasis or from normal individuals and can be treated with a variety of cytokines and/or growth factors to result in psoriatic phenotypes in this reconstituted human epidermal culture model of psoriasis. The organotypic model exhibits many features of human psoriasis including the upregulation of chemokines, induction of hyperproliferation, upregulation of S100 family members, and activation of phosphorylated signal transducer and activator of transcription (pStat3), one of the major signal transducers in psoriatic epidermis. This model can be useful for studying many aspects of the psoriatic epidermis, including keratinocyte differentiation and response to treatment stimuli.

### EXAMPLES

The following examples are offered by way of illustration and not by way of limitation.

### Example 1

Mice overexpressing a K14 driven hyperactive V12 Rac1 mutant display clinical-histologic features mimicking human psoriasis including hyperkeratosis, erythema, Koebnerization, Auspitz sign, arthritis, onychodystrophy, marked psoriasiform hyperplasia, parakeratosis, Munro microabcesses, dilated rete ridge blood vessels and characteristic immune infiltrates. Rac1 mouse and human psoriasis skin show similar epidermal thickness, suprabasilar Ki67, and increased expression of IL 1, 6, 17, 23, 36/CCL 2, 5, 17, 20/TNFa/TGFa/CARD14/IFIH1/IRFs, NFKB, STAT3 activation/β defensins/Act1/IL17R. Transcriptional comparison of psoriasis susceptibility genes in Rac1 mouse and human psoriatic skin show marked overlap of both epidermal and immune pathways. Human psoriasis lesional skin samples showed high epidermal Rac1 activation using Rac1-GTP mAb compared to age/location matched controls. A comparison of the mouse model with human psoriasis cells/tissue shows that epidermal Rac1 hyperactivation is a key event in psoriasis pathogenesis, which drives psoriasis pathways through the promotion of an abnormal epidermal-immune feedback loop. See, for example Winge et al. Journal of Investigative Dermatology (2014) 134, S9-S27.

Benzamil attenuates Rac1-mediated psoriasis in a mouse model. In a K14 Rac1 animal model for psoriasis, Benzamil was injected ip every other day for 0, 10 and 20 injections of (2mg/kg), or PBS. The Benzamil treated animal showed reduced scaling, erythema, edema and hyperkeratosis. The effect of Benzamil on epidermal thickness is dose dependent (2.1 / 1.4 / 0.7 mg/kg) and distinct from Amiloride (right). The treated skin was analyzed for mRNA transcripts, and showed a normalization of TNFα, s100a7 and k16, although not IL17 and IL22. Also, Benzamil treated skin display abolished suprabasal Ki67 protein expression, and strongly reduced epidermal TGFβ expression, whereas comparable, elevated numbers of CD3 and Cd11c+ immune cells are detected in both Benzamil treated and PBS treated skin.

Benzamil also reduces proliferation in primary human V12 Rac1 overexpressing keratinocytes and primary human psoriatic keratinocytes. V12 Rac1 keratinocytes display significantly increased proliferation compared to LacZ control keratinocytes. Benzamil significantly reduces proliferation in V12 Rac1 keratinocytes as well as in human psoriatic keratinocytes. Organotypic 3D skin equivalents seeded with V12 Rac1 or LacZ control keratinocytes were grown for 7 days at air-fluid interphase in the absence or presence of IL17 as well as without or with 10 µM Benzamil. IL17-stimulated Rac1 skin equivalents display reduced epidermal thickening in the presence of Benzamil.

Benzamil alters the subcellular localization of Rac1-GTP and reduces signaling pathways altered in human psoriasis. Benzamil treated skin display altered subcellular localization and reduced suprabasal expression of Rac1-GTP in a mouse model of psoriasis; reduced activated (nuclear translocation, phosphorylation) STAT3 and NFKB, but not altering interferon regulatory factors. Benzamil alters the subcellular localization of Rac1 in primary human psoriatic keratinocytes. In psoriasis keratinocytes, cytokine-mediated nuclear translocation of PSTAT3 and NFκB is reduced following Benzamil treatment. Benzamil alters the intracellular distribution of reactive oxygen species (ROS) and reduces total levels of ROS following IL17 stimulation.

Topical delivery of Benzamil in vivo with a liquid or solid formulation was effective in reducing scaling, erythema and epidermal thickening, as shown in Figure 3.

In vitro assays demonstrated that Benzamil targets proliferation in psoriatic keratinocytes. There was a significant decrease in psoriatic keratinocytes proliferation at 500 nM Benzamil concentrations. Comparison of mRNA expression of benzamil targets in a panel of human psoriasis lesional and non-lesional biopsies compared to control skin identified significant differences in both aENAC and NCX1. Results are shown in Figure 5.

The benzamil targets ENAC and NCX1 in human psoriatic keratinocytes. siRNA knockdown of aENAC upregulated NCX1 expression and siRNA knockdown of NCX1 upregulated aENAC expression. Benzamil restored the intrinsic Ca²⁺ buffering capacity of psoriatic keratinocytes, which was mimicked by sequential knockdown of both aENAC and NCX1.

These results were also replicated in an organotypic 3D in vitro model of psoriasis, where the effects of Benzamil were mimicked by sequential knockdown of benzamil targets aENAc and NCX1. Through isolation of primary keratinocytes and autologous fibroblasts, seeded on devitalized dermis and grown in air-fluid interphase, cytokine stimulation (IL23 25ng/ml;, IL17A/F 100ng/ml; TNFa 100ng/ml and IL22 25ng/ml) induced psoriasform hyperplasia, Rac1GTP expression and basal and suprabasal proliferation (ki67) in psoriasis cells, which was rescued by either Benzamil treatment (10uM) or sequential knockdown of aENAC and NCX1 (a). In contrast, conditions with control cells did not induce psoriasiform hyperplasia upon cytokine stimulation. The results are shown in Figure 7.

The dosages administered to animals and in vitro are shown below in Table 1.

**In vivo**

| | Route | IP | | | | |
|---|---|---|---|---|---|---|
| Vehicle | Dose (mg/kg) | Interval | Doses | Duration (days) | Effect | Assay |
| ddH20 | 10 | Daily | 20 | 20 | Full | In vivo |
| ddH20 | 2 | EOD | 20 | 40 | Full | In vivo |
| ddH20 | 1.4 | EOD | 20 | 40 | Partial | In vivo |
| ddH20 | 0.7 | EOD | 20 | 40 | Reduced | In vivo |

| | | | | | | |
|---|---|---|---|---|---|---|
| *EOD=every other day | | | | | | |

| | Route | Topical | | | | |
|---|---|---|---|---|---|---|
| Vehicle | Dose (mg/ml) | Interval | | | | |
| Vaseline | 2 | Daily | 20 | 20 | Partial | In vivo |
| ETOH | 2 | Daily | 20 | 20 | Full | In vivo |

**In vitro**

| | Route | In 50/50 KGM/K-154 media or KGM | | | | |
|---|---|---|---|---|---|---|
| Vehicle | Dose (uM) | Interval (h) | | Duration (24h days) | Effect | Assay |
| ddH20 | 0.1 | 24 | 1 | 1 | Low | WB |
| ddH20 | 1 | 24 | 1 | 1 | Low | WB |
| ddH20 | 10 | 24 | 1 | 1 | High | WB |
| ddH20 | 20 | 24 | 1 | 1 | High | WB |
| ddH20 | 50 | 24 | 1 | 1 | High | WB |
| ddH20 | 10 | 24 | 1 | 1 | High | Confocal IF cells |
| ddH20 | 1 | 24 | 1 | 1 | High | MTT proliferation |
| ddH20 | 10 | 24 | 1 | 1 | High | MTT proliferation |
| ddH20 | 50 | 24 | 1 | 1 | High | MTT proliferation |
| ddH20 | 0.05 | 24 | 1 | 1 | Low | MTT proliferation |
| ddH20 | 0.5 | 24 | 1 | 1 | High | MTT proliferation |
| ddH20 | 2.5 | 24 | 1 | 1 | High | MTT proliferation |
| ddH20 | 5 | 24 | 1 | 1 | High | MTT proliferation |
| ddH20 | 10 | 24 | 1 | 1 | High | MTT proliferation |
| ddH20 | 50 | 24 | 1 | 1 | High | MTT proliferation |
| ddH20 | 100 | 24 | 1 | 1 | High | MTT proliferation |
| ddH20 | 10 | 24 | 7 | 7 | High | Organotypic skin |

## Claims

1. A composition comprising benzyl amiloride for use in a method of treating psoriasis in a subject, wherein the method comprises administering to the subject an effective dose of benzyl amiloride for duration and periodicity sufficient to reduce one or more symptoms of psoriasis.

2. The composition for use of claim 1, wherein the administration is systemic.

3. The composition for use of claim 1, wherein the administration is topical.

4. The composition for use of any one of claims 1-3, wherein the dose is administered to the subject upon a flare of psoriasis.

5. The composition for use of any one of claims 1-4, wherein the psoriasis is chronic psoriasis.

6. The composition for use of any one of claims 1-4, wherein the psoriasis is plaque psoriasis.

7. The composition for use of any one of claims 1-6, wherein the composition further comprises a second therapeutic agent.

8. The composition for use of claim 7, wherein the second therapeutic agent is useful for the treatment of psoriasis.

9. The composition for use of any one of claims 1-8, wherein administration of the composition reduces one or more thickened cutaneous psoriatic plaques in the subject.

10. The composition for use of any one of claims 1 or 3-9, wherein the benzyl amiloride is administered at a concentration of 0.01 mg/m² to 100 mg/m² surface area of the subject, wherein the subject is an adult human.

11. The composition for use of any one of claims 1-10, wherein the benzyl amiloride is administered at 10-500 mg/day.

12. The composition for use of any one of claims 1-10, wherein the benzyl amiloride is administered at 1-110 mg/day.

## Patentansprüche

1. Zusammensetzung, die Benzylamilorid umfasst, zur Verwendung in einem Verfahren zur Behandlung von Psoriasis bei einem Individuum, wobei das Verfahren das Verabreichen einer wirksamen Dosis von Benzylamilorid an das Individuum für eine Dauer und mit einer Periodizität umfasst, die ausreichend sind, um eines oder mehrere Symptome von Psoriasis zu reduzieren.

2. Zusammensetzung zur Verwendung nach Anspruch 1, wobei die Verabreichung systemisch erfolgt.

3. Zusammensetzung zur Verwendung nach Anspruch 1, wobei die Verabreichung topisch erfolgt.

4. Zusammensetzung zur Verwendung nach einem der Ansprüche 1 bis 3, wobei die Dosis bei einem Psoriasisschub an das Individuum verabreicht wird.

5. Zusammensetzung zur Verwendung nach einem der Ansprüche 1 bis 4, wobei die Psoriasis chronische Psoriasis ist.

6. Zusammensetzung zur Verwendung nach einem der Ansprüche 1 bis 4, wobei die Psoriasis Plaque-Psoriasis ist.

7. Zusammensetzung zur Verwendung nach einem der Ansprüche 1 bis 6, wobei die Zusammensetzung weiters ein zweites Therapeutikum umfasst.

8. Zusammensetzung zur Verwendung nach Anspruch 7, wobei das zweite Therapeutikum zur Behandlung von Psoriasis geeignet ist.

9. Zusammensetzung zur Verwendung nach einem der Ansprüche 1 bis 8, wobei die Verabreichung der Zusammensetzung eine oder mehrere kutane psoriatische Plaque-Verdickungen bei dem Individuum reduziert.

10. Zusammensetzung zur Verwendung nach einem der Ansprüche 1 oder 3 bis 9, wobei das Benzylamilorid in einer Konzentration von 0,01 bis 100 mg/m² Oberfläche des Individuums verabreicht wird, wobei das Individuum ein adulter Mensch ist.

11. Zusammensetzung zur Verwendung nach einem der Ansprüche 1 bis 10, wobei 10 bis 500 mg/Tag Benzylamilorid zu verabreichen sind.

12. Zusammensetzung zur Verwendung nach einem der Ansprüche 1 bis 10, wobei 1 bis 110 mg/Tag Benzylamilorid zu verabreichen sind.

## Revendications

1. Composition comprenant du benzylamiloride destinée à être utilisée dans un procédé de traitement du psoriasis chez un sujet, le procédé comprenant l'administration au sujet d'une dose efficace de benzylamiloride pendant une durée et une périodicité suffisantes pour réduire un ou plusieurs symptômes du psoriasis.

2. Composition pour utilisation selon la revendication 1, dans laquelle l'administration est systémique.

3. Composition pour utilisation selon la revendication 1, dans laquelle l'administration est topique.

4. Composition pour utilisation selon l'une quelconque des revendications 1 à 3, dans laquelle la dose est administrée au sujet lors d'une poussée de psoriasis.

5. Composition pour utilisation selon l'une quelconque des revendications 1 à 4, dans laquelle le psoriasis est un psoriasis chronique.

6. Composition pour utilisation selon l'une quelconque des revendications 1 à 4, dans laquelle le psoriasis est un psoriasis en plaques.

7. Composition pour utilisation selon l'une quelconque des revendications 1 à 6, dans laquelle la composition comprend en outre un second agent thérapeutique.

8. Composition pour utilisation selon la revendication 7, dans laquelle le second agent thérapeutique est utile pour le traitement du psoriasis.

9. Composition pour utilisation selon l'une quelconque des revendications 1 à 8, dans laquelle l'administration de la composition réduit une ou plusieurs plaques psoriasiques cutanées épaissies chez le sujet.

10. Composition pour utilisation selon l'une quelconque des revendications 1 ou 3 à 9, dans laquelle le benzylamiloride est administré à une concentration de 0,01 mg/m² à 100 mg/m² d'aire de surface du sujet, dans laquelle le sujet est un être humain adulte.

11. Composition pour utilisation selon l'une quelconque des revendications 1 à 10, dans laquelle le benzylamiloride est administré à raison de 10 à 500 mg/jour.

12. Composition pour utilisation selon l'une quelconque des revendications 1 à 10, dans laquelle le benzylamiloride est administré à raison de 1 à 110 mg/jour.
